(19) **Europäisches Patentamt**
**European Patent Office**
**Office européen des brevets**

(11) **EP 2 009 096 B1**

(12) **EUROPÄISCHE PATENTSCHRIFT**

(45) Veröffentlichungstag und Bekanntmachung des Hinweises auf die Patenterteilung:
**09.11.2016 Patentblatt 2016/45**

(21) Anmeldenummer: **07747891.5**

(22) Anmeldetag: **04.04.2007**

(51) Int Cl.:
**C12N 5/0775** (2010.01)

(86) Internationale Anmeldenummer:
**PCT/RU2007/000178**

(87) Internationale Veröffentlichungsnummer:
**WO 2007/114740 (11.10.2007 Gazette 2007/41)**

(54) **BIOTRANSPLANTAT FÜR DIE ZELLTHERAPIE AUF DER GRUNDLAGE MESENCHYMALER KNOCHENMARK-STAMMZELLEN**

BIOTRANSPLANT FOR CELLULAR THERAPY BASED ON MESENCHYMAL BONE MARROW STEM CELLS

TRANSPLANT BIOLOGIQUE DESTINÉ À LA THÉRAPIE CELLULAIRE À BASE DE CELLULES SOUCHES MÉSENCHYMATEUSES, OBTENUES À PARTIR DE LA MOELLE OSSEUSE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **04.04.2006 RU 2006110590**
**23.05.2006 RU 2006117602**
**23.05.2006 RU 2006117609**

(43) Veröffentlichungstag der Anmeldung:
**31.12.2008 Patentblatt 2009/01**

(73) Patentinhaber: **Trans-Technologies Ltd.**
**St. Petersburg 189650 (RU)**

(72) Erfinder:
• **BUHARTSEV, Nikolay Nikolaevich**
**St.Petersburg, 191002 (RU)**
• **VIJDE, Svetlana Nikolaevna**
**St.Petersburg, 198206 (RU)**
• **GALANIN, Igor Veniaminovich**
**St.Petersburg, 193029 (RU)**
• **KISLJAKOVA, Tatyana Vitaljevna**
**St.Petersburg, 191002 (RU)**
• **KRUGLAKOV, Peter Vladimirovich**
**Leningradskaya obl., 188694 (RU)**
• **POLYNTSEV, Dmitry Genrihovich**
**St.Petersburg, 197349 (RU)**
• **SOKOLOVA, Irina Borisovna**
**Leningradskaya obl., 188680 (RU)**
• **SKOROMETS, Taras Aleksandrovich**
**St.Petersburg, 198206 (RU)**

(74) Vertreter: **Stanjek, Antje Monika**
**Kador & Partner**
**Corneliusstrasse 15**
**80469 München (DE)**

(56) Entgegenhaltungen:
EP-A- 1 767 617    WO-A-00/69449
WO-A-03/070922    WO-A-2005/121317

• KAN I ET AL: "Integral therapeutic potential of bone marrow mesenchymal stem cells." CURRENT DRUG TARGETS FEB 2005, vol. 6, no. 1, February 2005 (2005-02), pages 31-41, XP002456405 ISSN: 1389-4501
• WAGNER ET AL: "Comparative characteristics of mesenchymal stem cells from human bone marrow, adipose tissue, and umbilical cord blood" EXPERIMENTAL HEMATOLOGY, NEW YORK, NY, US, vol. 33, no. 11, November 2005 (2005-11), pages 1402-1416, XP005136979 ISSN: 0301-472X
• STUTE N ET AL: "Autologous serum for isolation and expansion of human mesenchymal stem cells for clinical use" EXPERIMENTAL HEMATOLOGY, NEW YORK, NY, US, vol. 32, no. 12, December 2004 (2004-12), pages 1212-1225, XP004674871 ISSN: 0301-472X

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- CHEN X ET AL: "HUMAN BONE MARROW STROMAL CELL CULTURES CONDITIONED BY TRAUMATIC BRAIN TISSUE EXTRACTS: GROWTH FACTOR PRODUCTION" JOURNAL OF NEUROSCIENCE RESEARCH, WILEY-LISS, US, vol. 69, no. 5, 2 August 2002 (2002-08-02), pages 687-691, XP009044236 ISSN: 0360-4012
- JUN FENG JI ET AL: "Interactions of chemokines and chemokine receptors mediate the migration of mesenchymal stem cells to the impaired site in the brain after hypoglossal nerve injury" STEM CELLS, ALPHAMED PRESS, DAYTON, OH, US, vol. 22, no. 3, May 2004 (2004-05), pages 415-427, XP009055260 ISSN: 1066-5099
- PITTENGER M F ET AL: "MULTILINEAGE POTENTIAL OF ADULT HUMAN MESENCHYMAL STEM CELLS" SCIENCE, AMERICAN ASSOCIATION FOR THE ADVANCEMENT OF SCIENCE,, US, vol. 284, no. 5411, 2 April 1999 (1999-04-02), pages 143-147, XP000867221 ISSN: 0036-8075 -& PITTENGER M F; ET AL: "MULTILINEAGE POTENTIAL OF ADULT HUMAN MESENCHYMAL STEM CELLS" SCIENCE, [Online] vol. 284, 2 April 1999 (1999-04-02), pages 1-7, XP002456406 Retrieved from the Internet: URL:http://www.sciencemag.org/feature/data /983855.dtl> [retrieved on 2007-10-24]
- SOKOLOVA I B ET AL: "Effect of mesenchymal stem cell transplantation on cognitive functions in rats with ischemic stroke" BULLETIN OF EXPERIMENTAL BIOLOGY AND MEDICINE, KLUWER ACADEMIC PUBLISHERS-PLENUM PUBLISHERS, NE, vol. 142, no. 4, 1 October 2006 (2006-10-01), pages 511-514, XP019463877 ISSN: 1573-8221
- SOKOLOVA I B ET AL: "Distribution of mesenchymal stem cells in the area of tissue inflammation after transplantation of the cell material via different routes" BULLETIN OF EXPERIMENTAL BIOLOGY AND MEDICINE, KLUWER ACADEMIC PUBLISHERS-PLENUM PUBLISHERS, NE, vol. 143, no. 1, 1 January 2007 (2007-01-01), pages 143-146, XP019497926 ISSN: 1573-8221

**Beschreibung**

Die Erfindung bezieht sich auf ein Verfahren zur Gewinnung einer Population von Mesenchymstammzellen nach dem Oberbegriff des Anspruchs 1.

Die Erfindung ist auf dem Gebiet der Biotechnologie und der Biopharmakologie einsetzbar und befasst sich mit der Erzeugung von Biotransplantat aus dem Knochenmark des Menschen auf der Basis von Mesenchymstammzellen der homogenen Zellenpopulation. Die Erfindung kann in der Medizin zur Behandlung von verschiedenen Krankheiten angewendet werden. Bei Einsatz von Knochenmark von Tieren (der Säugetiere) gemäß der Erfindung kann sie in der Tierheilkunde angewendet werden.

Die Erfindung kann auch in der klinischen Psychiatrie bei der Behandlung von Schädigungen der intellektuellen und mnestischen Funktionen mit unterschiedlicher Ätiologie angewendet werden, sowie bei der Behandlung von pathologisch veränderten Gehirngeweben, die mit einem Schädel-Hirn-Trauma oder mit akuten Änderungen des Gehirnkreislaufs verbunden sind.

Heutzutage gewinnt die Zellulartherapie sehr viel an Bedeutung. Eines der modernen und aktiv entwickelten Verfahren der Behandlung von verschiedenen therapeutisch resistenten Schädigungen des Zentralnervensystems (ZNS) ist die intrakraniale Transplantation der Mesenchymstammzellen (MSZ). Diesem vielversprechenden Verfahren der Wiederherstellung der gestörten Funktionen des Zentralnervensystems liegen nicht nur die Möglichkeiten der gezielten Einwirkung auf bestimmte funktionelle Strukturen des Gehirns sondern auch der normalisierende Einfluss auf Imbilanz der neuroübertragenden Gehirnsysteme zugrunde. Dies ist tatsächlich ein grundsätzlich neuer Ansatz bei der Behandlung einer großen Gruppe von Erkrankungen, die mit Schädigungen des ZNS zusammenhängen.

Die Stammzellen umfassen:

- die Mesenchymzellen; sie sind fähig, sich in die Zellen der Gewebe der mesodermalen Herkunft zu differenzieren,
- die hämopoetischen Zellen (Vorgänger der Blutzellen),
- die Neuronalzellen (Vorgänger der Zellen des Nervensystems) und andere Zellen.

Die Mesenchymstammzellen (MSZ) werden als Biotransplantat benutzt, weil sie die eigenen Körperressourcen aktivieren und zur Erzeugung von Zytokinen und der Wachstumsfaktoren beitragen. MSZ sind die Vorgänger der Osteoblaste und tragen zur Bildung von remodelierenden Einheiten bei.

Die MSZ werden aus fetalen Blutbildungsorganen, aus Skelettmuskel, aus subkutanem Fettgewebe oder aus Knochenmark gewonnen. Bei der Arbeit mit MSZ werden traditionelle Umgangsmethoden mit den Zellen (Tierzellenkultur. Methoden. Herausgegeben von R. Freshni. Moskau. Mir. 1989) eingesetzt. In jedem Fall werden ein individuell abgestimmter Wirkungsablauf, Absonderungsbedingungen und Bedingungen für die resultierende Zellenmasse gewählt.

Die MSZ für die Transplantation wird aus dem Spendermaterial, autologen oder fetalen Material gewonnen. Die Zellkultur kann im Voraus vorbereitet werden. Nach der Produzierung der Zellenmasse wird sie gemäß dem Kryokonservierungsverfahren gelagert.

Das beste Material für die Produzierung der transplantatgeeigneten MSZ ist das Fetalmaterial. Das liegt daran, dass die Zellen über eine ausgeprägte Differenzierungsfähigkeit verfügen, funktionsmäßig aktiver sind, ein großes Potential an Proliferation und Wachstum aufweisen und die Regenerierungs- und Wachstumfaktoren produzieren. Bei einer Verpflanzung regen diese Zellen die Angiogenese an. Die fetalen Zellen überstehen den Sauerstoffmangel besser, jedoch hängt die Nutzung der fetalen Zellen mit ethischen Problemen sowie mit einer zu geringen Zugänglichkeit des Ausgangsmaterials zusammen.

Es ist ein Verfahren zur Gewinnung von MSZ für die Implantation zwecks gezielter Osteoregeneration (Patent der RF Nr. 2210352 vom 20.08.2003) bekannt. Es wurde eine homogene (mindestens 80 %) Population von pluripotenten MSZ gezüchtet. Sie wurden mittels Disaggregation des Ursprungsgewebes von MSZ unter einer nachfolgender Neuaufschwemmung der Suspension und Züchtung auf dem Wachstumsnährboden erzeugt. Der Mangel dieser Methode ist die Erzeugung von undifferenzierten MSZ infolge der heterogenen Population.

Es ist ein Verfahren zur Gewinnung von Biotransplantat zur Behandlung von Osteoporose (Patent der RF Nr. 2265442 vom 10.12.2005) bekannt, welches aus MSZ besteht. Während der Gewinnung von MSZ werden enzymatische Disaggregation, Filterung durch ein feinmaschiges Filter, Zentrifugieren in der Hanks-Salzlösung und Neuaufschwemmung auf dem Wachstumsnährboden DMEM/F12 vorgenommen. Der Wachstumsnährboden enthielt 15 % embryonales Kalbsserum und Glutamin. Die Aussaat erfolgt auf Petrischalen aus Kunststoff. Die nicht angehafteten Zellen wurden 24 Stunden später entfernt. Die Inkubation erfolgte bei 37° C in der Atmosphäre mit einem $CO_2$-Gehalt von 5 %. Anschließend wurde eine Trypsinisierung und ein Überimpfen der Kultur vorgenommen. Infolge einiger Überimpfungen wurde die Zellkultur MSZ mit der vorwiegenden Fähigkeit zur osteogenen Differenzierung gewonnen. Diese Kultur stellt jedoch die heterogene Zellpopulation dar, der keine Phänotypisierung eigen ist.

Unterschiedliche Quellen für die Gewinnung von MSZ setzen auch unterschiedliche Verarbeitung des biologischen Ausgangsmaterial voraus: Fettgewebe, Nabelschnurblut, Fetalmaterial, Knochenmark. Die Bedingungen zur Aussonderung von MSZ für weitere Züchtung sowie die Bedingungen für die Speicherung der erforderlichen Zellmasse hängen von der Zusammensetzung des Kulturbodens, den Aussaatbedingungen, der Züchtungsbedingungen, vom Geschirr und anderen Faktoren ab.

Es ist ein Verfahren zur Gewinnung von MSZ bekannt, welches auf der Fähigkeit der Zellen beruht, an der Oberfläche des Geschirrs für die Züchtung der Zellkultur zu haften (Bone, 1995, vol. 13, p. 81-95). Während des Vorgangs wird das embryonale Ochsenserum (nämlich ihre ausgewählten Lose) genommen. Es wurden Zellen mit hoher Adhäsionsfähigkeit, hoher Proliferationsgeschwindigkeit und dauerhafter Multipotenzerhaltung gewonnen. Die Mängel dieses Verfahrens umfassen die Arbeitsaufwändigkeit bei der Analyse des Ochsenserums, welches für die Zellzüchtung angewendet wird, sowie die sehr geringe Reproduzierbarkeit des Verfahrens. Deswegen wird eine große Streuung der Ergebnisse der MSZ-Gewinnung festgestellt.

Die Aussonderung und die Züchtung der Mesenchymstammzellen sind verhältnismäßig unkompliziert. Es besteht jedoch das Problem im Zusammenhang mit der Erzeugung einer homogenen Zellpopulation von MSZ, diese in einer solchen Menge zu erhalten, die für die Transplantation und die Konservierung für eine spätere Nutzung ausreichend ist.

Es ist ein Verfahren zur Aussonderung der Mesenchymstammzellen nach der internationalen Anmeldung WO2005121317 (Priorität nach dem Patent der RF Nr. 2252252 vom 20.05.2005) bekannt. Nach diesem Verfahren werden die Gewebe des Menschen zerkleinert, mit Kollagenase-Lösung im Eagle-Nährboden nach der Dulbeccos-Modifizierung behandelt und zentrifugiert. Danach werden die Erythrozyten aus der erzeugten Suspension mittels der lysierenden Lösung mit nachfolgender Filterung durch Filter mit unterschiedlichen Maschengrößen entfernt. Die Zellen werden mittels Aussaat in Flaschen gezüchtet. Es werden lebensfähige homogene Zellen mit einer hohen Ausbeute erzeugt. Die Ausbeute beträgt $10^6$ / 1 g des Gewebes. Der Mangel dieser Methode ist die Mehrstufigkeit der Reinigung. Dies ist auf das Ausgangsmaterial für die Aussonderung von MSZ zurückzuführen, denn es handelt sich um Fettgewebe oder Plazenta. Nur bei der 4. Passage erhalten die Urheber die homogenen Zellen, welche dem Immun-Phänotyp MSZ entsprechen: CD 13+, CD 44+, CD 90+, CD105-, CD 31-, CD 45-, CD 117-.

Nach den bekannten Verfahren der MSZ-Züchtung aus Knochenmark werden normalerweise heterogene Zellpopulationen Stroma mit geringem Stammzellengehalt darin erzeugt.

Es ist ein Werk von Majumdar M. K. et al. (J. Cell. Physiol., 2000, Oct, 185(1), p. 98-106) bekannt. Hier werden die Aussonderung, die Charakteristik und das Chondrogenenpotential der stromogenen Multiplett-Zellen betrachtet, welche aus Knochenmark des Menschen erzeugt werden. Die Zellen werden unter Einsatz von Endoglin-Expression ausgesondert. Sie wurden in vitro in einem Medium ohne Serum unter Anwesenheit von einem Transformationswachstumfaktor-Beta (TGF-beta) gezüchtet. Dabei werden für das Wachstum dreidimensionale Matrizen von Alginatkugeln angewendet. Die Zellen werden nur durch den CD 34+ Marker gekennzeichnet.

Es ist ein Verfahren zur Gewinnung von MSZ aus Knochenmark des Menschen bekannt, wonach mononukleare Zellen mittels Zentrifugierung im Ficoll-Gradienten unter nachfolgender Selektion in Bezug auf Antikörper gegenüber Oberflächenantigen CD 105+ abgeleitet werden. Dieses Oberflächenantigen zeigt seine Expression an der Oberfläche von MSZ. Die Zellen werden je nach Adhäsion der MSZ zum Kunststoff gewählt. Danach werden die Zellen gezüchtet (J. Cell. Physiol. 2000. vol. 185. p. 98-106). Im Endeffekt wird eine Zellfraktion CD 105+ abgeleitet, welche mit MSZ angereichert ist. Die Ausbeute der Zellen ist nicht hoch, während das Verfahren selbst sich als ziemlich aufwändig erweist.

Die aus dem Knochenmark des Rezipienten gewonnene Zellmasse enthält (im Punktat) max. 1-3 % Stammzellen, d.h. Zellen mit polypotenten Eigenschaften. Der Hauptanteil des Punktats macht bei Entnahme das periphere Blut aus. Um das Biotransplantat zu erhalten, sind Methoden zur Speicherung der phänotypmäßig homogenen MSZ-Masse erforderlich. Diese Masse sollte für medizinische Zwecke geeignet sein und in der zur Behandlung erforderlichen Menge (80-100 Mio. Zellen pro Prozedur) gezüchtet werden. Die weiteren Voraussetzungen für die Gewinnung sind minimaler Zeit- und Geldaufwand sowie minimale Verletzung des Rezipienten.

Die Quelle der MSZ spielt eine wesentliche Rolle in der nachfolgenden Verwendung der Zellen bei der Krankenbehandlung (Transplantation der hämopoetischen Stammzellen, A.G. Rumyantsev, A.A. Maschan - Medizinische Infoagentur. M. - 2003 - 912 S.)

Die Behandlung der Affektion der intellektuellen und mnestischen Funktionen hängt mit der Beanspruchung des zentralen Nervensystems (ZNS), der Hirnrinde, zusammen.

Es ist eine Methode der Neurotransplantation der embryonalen Stammzellen (NESZ) bekannt, welche bei unterschiedlichen therapeutisch resistenten Schädigungen von ZNS angewendet wird (A.S. Brukhovetsky, Nervenzellentransplantation und Gewebe-Hirnengineering bei Nervenkrankheiten, M. 2003, S. 398., V.V. Semchenko und andere. Neurotransplantation, Omsk, 2004, S. 306.) (2, 3).

Weiter ist ein Verfahren zur Behandlung von psychischen Sprech- und Entwicklungshemmungen unterschiedlicher Herkunft mittels Neurotransplantation des embryonalen Nervengewebes in die Hirnrinde (RU, Patent Nr. 2219937, 2003) bekannt.

Es ist ein Verfahren zur Behandlung von neurodegenerativen Erkrankungen mittels einmaliger endolumbaler Einführung von 50-500 Mio. neuronaler Stammzellen (NSZ) in 2 ml der physiologischen Lösung (Patent der RF Nr.

2259836 vom 10.09.2005) bekannt. Die Kultur der genetisch unmodifizierten NSZ wurde aus embryonalem Hirngewebe erzeugt. Das Zell-Biotransplantat stellt die Funktion des beschädigten Gehirns wieder her und verbessert sie. Die eingeführten Zellen sind unhomogen, unterschiedlich ihrem Phänotyp nach und werden mittels Lymphe zu den beschädigten Stellen befördert.

Es ist ein Verfahren zur Behandlung von zerebrovaskulärem Insult bekannt. Es werden dem Patienten 50 bis 500 Mio. neuronaler Stammzellen (NSZ) verabreicht, welche aus dem Embryogehirngewebe gewonnen wurden. Die NSZ werden in 2 ml der physiologischen Lösung endolumbal eingeführt. Das bekannte Biotransplantat besteht aus NSZ in der physiologischen Lösung. Der Mangel dieses Verfahrens liegt im Einsatz von undifferenzierten Zellen. Es besteht auch ein ethisches Problem im Zusammenhang mit der Herkunft von NSZ (Patent der RF Nr. 2258521 vom 20.08.2005).

Es ist ein Verfahren zur Behandlung von Gehirnschädigungen mittels intrazerebraler Einführung der hämopoetischen Stammzellen bekannt (Patent der RF Nr. 2216336 vom 20.11.2003). Das Ergebnis der Behandlung nach dieser bekannten Methode beruht darauf, dass die hämopoetischen Stammzellen nach der Transplantation in die betroffene Gehirnstelle auf die Signale dieser Gehirnstelle reagieren. Dabei zeigen sie einen solchen Phänotyp, welcher die durch die genannte Schädigung oder Erkrankung verursachte funktionelle Insuffizienz ersetzen oder ausgleichen kann. Nach dieser Methode werden undifferenzierte Zellen benutzt, die nicht unbedingt eine abgeleitete Form aufweisen, weil das Zellmaterial phänotypmäßig nicht homogen ist.

Bekanntermaßen werden bei der Neurotransplantation als Spendermaterial Embryogewebe, insbesondere die embryonalen Neuroblaste des Menschen, angewendet. Im Unterschied dazu werden die MSZ nach diesem Verfahren aus Knochenmark des Patienten selbst (Autotransplantation) gewonnen. Dies ermöglicht es, eine ganze Reihe von Problemen zu vermeiden.

Vor allem wird das Problem der Bluthirn- und Immunreaktionen vollständig beseitigt, weil dem Patienten seine eigenen Zellen verpflanzt werden. Dabei verursachen die autoimmunen Zellen keine Abstoßungsreaktionen, sie verfügen umgekehrt über noch mehr immunosuppressive Eigenschaften.

Bei der Anwendung der Autotransplantation der MSZ sind auch religiöse, ethische und rechtliche Probleme, die die Anwendung von allogenen Neuroblasten immer begleiten, nicht mehr relevant. Die allogenen Neuroblaste erschweren wesentlich den Einsatz von embryonalen Geweben oder der daraus gewonnenen Zellkulturen.

Darüber hinaus werden die organisatorischen Probleme beachtlich vereinfacht. Diese Probleme hängen normalerweise damit zusammen, dass es unbedingt notwendig ist, die Spendergewebe in Bezug auf zahlreiche Virusinfektionen oder Erbleiden zu untersuchen.

Die MSZ sind Vorgänger-Zellen. Daraus werden im Laufe der weiteren Spezialisierung entweder Nervenzellen oder Zellen für unterschiedliche Gewebearten herausgebildet. Die "Spezialisierung" der MSZ hängt von der unmittelbaren Zellumgebung ab, in die die MSZ gelangen. Somit eignen sich die unmittelbar ins Gehirngewebe verpflanzten oder ins Gehirn über das Gefäßsystem eingeführten MSZ alle funktionellen Besonderheiten der sie umgebenen Neurone an. Nachfolgend heilt sich ein Teil von diesen MSZ ein, differenziert und stellt Verbindungen zu anderen Umgebungsnervenzellen her. Diese MSZ üben einen normalisierenden Einfluss auf die Imbilanz der neuroübertragenden Systeme des kranken Gehirns aus. Dies stellt einen grundsätzlich neuen Ansatz bei der Behandlung einer ganzen Reihe von Erkrankungen dar, welche mit den Affektionen des ZNS zusammenhängen.

Es ist Aufgabe der Erfindung, ein Verfahren zur Gewinnung einer Population von Mesenchymstammzellen (MSZ) mit minimalem Zeit-, Energie- und Materialaufwand bereitzustellen.

Die gestellte Aufgabe wird durch die Merkmale des Anspruchs 1 gelöst.

Die gewonnenen Mesenchymstammzellen aus Knochenmark können Basis für ein Biotransplantat für die Zellulartherapie sein, das nicht im Rahmen der Erfindung liegt. Das Biotransplantat auf der Grundlage der Mesenchymstammzellen der homogenen Zellenpopulation kann zur Erhöhung der Behandlungseffektivität bei einer ausgeprägten intellektuellen und mnestischen Funktionsleistungsabnahme im Zusammenhang mit mangelnder psychischer Entwicklung oder zur Korrektur der sensomotorischen und/oder kognitiven Insuffizienz infolge eines Schädelhirntraumas oder der akuten Störung des Gehirnkreislaufs angewendet werden. Dabei werden die Mesenchymstammzellen aus Knochenmark des Menschen verschiedenen Alters abgeleitet.

Das Biotransplantat zum therapeutischen Einsatz stellt eine Suspension dar. Diese Suspension enthält als wirksamen Bestandteil die lebensfähige Population der Mesenchymstammzellen und einen pharmazeutisch geeigneten Träger. Die Population der Mesenchymstammzellen ist zu 87-99,9 % nach den Zellmarkern CD 44+, CD 90+, CD105+, CD106+, CD 45-, CD 34- homogen und wird aus dem Knochenmark der Säugetiere hergestellt.

Weitere zweckmäßige und vorteilhafte Maßnahmen der Erfindung gehen aus den Unteransprüchen hervor.

Das Knochenmark wird aus dem Brustbein und/oder aus der Darmbeinschaufel des Beckens gewonnen. Dabei kann das Knochenmark autolog sein oder von einem Spender stammen.

Das Biotransplantat enthält eine frisch aufbereitete Population der Mesenchymstammzellen.

Das Biotransplantat kann eine konservierte Population der Mesenchymstammzellen enthalten. Insbesondere wird als Kryoschutzmittel das Dimethylsulfoxid eingesetzt.

Das erfindungsgemäße Verfahren dient der Gewinnung von einer Population der Mesenchymstammzellen (MSZ) mit phänotypmäßiger Homogenität zu 87-99,9 % nach den Zellmarkern CD 44+, CD 90+, CD105+, CD106+, CD 45-, CD 34- aus Knochenmark der Säugetiere. Das Verfahren umfasst die Ableitung von MSZ mittels der Zentrifugierung und ihre Züchtung im Kulturnährboden. Die lebensfähigen Zellen werden an der Grenze der Medien DMEM - Ficoll in der 2. Stufe der Verarbeitung des Ausgangsmaterials nach einer 25 bis 60 Minuten langen Zentrifugierung der aufs Ficoll aufgeschichteten Suspension der Zellen des heparinisierten Knochenmarkpunktats im DMEM bei einer Geschwindigkeit von 1000 - 2000 U/Min. abgeleitet. Die Züchtung der Zellmasse erfolgt aus den gewonnenen MSZ im genormten Geschirr. Dabei wird das DMEM-Medium unter Zusatz von 15-22 % FBS eingesetzt. Dafür werden Zellen mit einer Dichte von 75-200 Zellen pro 1 cm$^2$ zugesetzt. Die Inkubationsdauer beträgt 18 bis 52 Stunden.

Die Zentrifugierung wird im Ficoll mit einer Dichte von 1,077 g/l oder 1,115 g/l bei einer Temperatur von 18-23° C vorgenommen.

Die Zellmasse in der Menge von 1,5 - 3 x 10$^6$ bis 1,5 - 3 x 10$^7$ Zellen pro 1 ml des Ausgangsknochenmarks werden im Laufe von drei Passagen gewonnen.

Das Biotransplantat wird auch zur Behandlung von Gehirnstörungen vorgeschlagen. In diesem Biotransplantat stellt die lebensfähige Population der zu 87-99,9 % nach den Zellmarkern CD 44+, CD 90+, CD 105+, CD 106+, CD 45-, CD 34- homogenen Mesenchymstammzellen (MSZ) die autologen MSZ dar, welche aus Knochenmark gewonnen werden. Diese MSZ sind in dem 10%-igen autologen Blutserum auf der Basis einer physiologischen Lösung oder im pharmazeutisch geeigneten Träger enthalten.

Das Biotransplantat enthält eine frisch aufbereitete Population der Mesenchymstammzellen.

Das Biotransplantat enthält eine kryokonservierte Population der Mesenchymstammzellen, insbesondere unter Einsatz von Dimethylsulfoxid als Kryoschutzmittel.

Die Konzentration der Mesenchymstammzellen beträgt 0,7 - 1,2 x 10$^6$ MSZ in 1 ml Biotransplantat.

Das Biotransplantat zur intrazerebralen Einführung enthält einen pharmazeutisch geeigneten Träger, insbesondere einen hämostatischen Schwamm.

Es wurde ein Verfahren zur Behandlung der Kranken mit ausgeprägter psychischer Unterentwicklung vorgeschlagen. Das Verfahren besteht darin, dass das Biotransplantat zur Behandlung von Gehirnstörungen in einer therapeutisch wirksamen Menge mittels eines pharmazeutisch geeigneten Trägers parenchymatös in eine oder mehrere Brodmann-Zonen 44, 43, 46, 24, 25, 22, 41 und 42 eingeführt wird.

Die Menge der eingeführten autologen Mesenchymstammzellen beträgt 7-12 Mio. pro Brodmann-Zone.

Das Biotransplantat zur Behandlung von Gehirnstörungen wird zusätzlich intravaskulär mit einer Dosierung von 0,5 - 0,7 x 10$^6$ der autologen Mesenchymstammzellen pro 1 kg des Patientengewichts eingeführt.

Das Verfahren zur Behandlung der Kranken mit einer ausgeprägten psychischen Unterentwicklung besteht darin, dass das Biotransplantat zur Behandlung von Gehirnstörungen intravaskulär mittels intraarterieller und/oder intravenöser Injektion in einer therapeutisch wirksamen Menge eingeführt wird.

Es wurde das Verfahren zur Behandlung von Folgeerscheinungen des Schädelhirntraumas und der akuten Störungen des Gehirnkreislaufs vorgeschlagen. Das Verfahren besteht darin, dass das Biotransplantat zur Behandlung von Gehirnstörungen parenchymatös mittels eines pharmazeutisch geeigneten Trägers und/oder intravaskulär in einer therapeutisch wirksamen Menge eingeführt wird.

Die intraarterielle Injektion des Biotransplantats erfolgt mit einer Geschwindigkeit von 2 ml/Min.

Die intravenöse Injektion des Biotransplantats erfolgt mit einer Geschwindigkeit von 6 ml/Min.

Die therapeutisch wirksame Menge von MSZ wird intrazerebral in die zur Behandlung ausgewählten Zonen der Hirnrinde eingeführt. Dabei beträgt die Menge der autologen Mesenchymstammzellen 7-12 Mio. pro Zone.

Die genannte Behandlungsweise ist für jedes Säugetier anwendbar, ist jedoch vorwiegend für die Behandlung von Menschen anwendbar.

Das technische Ergebnis des erfindungsgemäßen Verfahrens ist die Optimierung des Ablaufs bei der Ableitung der MSZ mit einer hohen Homogenität aus Knochenmark des Rezipienten zur Herstellung vom Biotransplantat sowie die Beschleunigung dieses Vorgangs. Die Vereinfachung und die Verbilligung der Erzeugung der MSZ-Kultur in den für die Gewinnung von Biotransplantat erforderlichen Mengen hängen mit der niedrigen Dichte der Zellenaussaat zusammen. Dadurch unterscheidet sich dieses Verfahren von den anderen bekannten Methoden. Außerdem entfällt in diesem Fall die Benutzung von Sondergeschirr und spezifischen Medien.

Die Bedingungen für die Gewinnung der MSZ-Kultur sind so ausgewählt, dass die Einwirkung der Chemikalien auf die Zellen minimiert wird, d.h. die Zellen werden keinem negativen Einfluss der Reagenzien ausgesetzt. Die nach dem erfindungsgemäßen Verfahren gewonnenen MSZ waren lebensfähig. Sie wurden durch 6 Marker nach dem Phänotyp CD 44+, CD 90+, CD105+, CD106+, CD 45-, CD 34- gekennzeichnet. Die Ausbeute der homogenen Zellen betrug bis zu 99,9 %.

Die Zellenausbeute beträgt von 1,5 - 3 x 10$^6$ bis 1,5 - 3 x 10$^7$ pro 1 ml des Ausgangsmaterials, d.h. des Knochenmarks. Somit wurde das Primärmaterial so vollständig wie möglich verbraucht, die Verluste wurden minimiert.

Es wurden optimale Züchtungsbedingungen für MSZ in vitro gewählt. Deswegen nimmt der Vorgang der Zellmassenspeicherung 7 bis 9 Tage in Anspruch. Die Anzahl der durchgeführten Prozeduren wurde minimiert.

Das Wesen dieser Erfindung ist die Ableitung von einer zu 80-99,9 % homogenen Zellfraktion in der 2. Stufe (die Selektionsstufe), nachdem die angereicherte MSZ-Kultur aus dem desaggregierten Knochenmark gewonnen wurde. Die Ableitung von MSZ erfolgt mittels Zentrifugierung im Laufe von 25-60 Minuten bei einer Geschwindigkeit von 1000-2000 U/Min im Ficoll. Die nachfolgende Züchtung der an der Mediengrenze gesammelten Zellpopulation erfolgt durch Subkultivierung in Kolben unter Überwachung des Saatguts in Bezug auf die sich ringsum bildenden Zellen und unter Überwachung der Vollständigkeit der Entnahme der Zellmasse, welche durch die Methoden der Immun-Phänotypisierung als MSZ gekennzeichnet ist. Der Kulturnährboden besteht aus dem Nährmedium DMEM mit 15- 22 % FBS.

Werden die Kennwerte bei der Durchführung der angemeldeten Abläufe (Vergrößerung oder Verminderung) geändert, so kann das genannte technische Ergebnis der Erfindung nicht mehr erreicht werden, weil dabei eine akute Abnahme der MSZ-Ausbeute entsteht und die Homogenität der Zellensuspension gestört wird.

In Bezug auf den therapeutischen Einsatz haben die autologen Zellen Vorrang, weil sie eine Fraktion der hochgereinigten eigenen Stammzellen darstellen. Die im Kulturnährboden gezüchteten autologen Mesenchymstammzellen (AMSZ) sind bei der Verabreichung für den Patienten gefahrlos, weil es sich um seine eigenen Zellen handelt. Dadurch wird die Möglichkeit von allergischen Reaktionen ausgeschlossen. Die große Menge der eingeführten Zellen ergibt eine ausgeprägte Heilwirkung.

Bei der Behandlung von Gehirnstörungen hängt die Transplantation der MSZ in die Hirnrinde mit ihrer Fähigkeit zusammen, die Funktion des unterentwickelten (affektierten) Gehirns zu verbessern. Die MSZ sind auch fähig, die Verbindungen in bestimmten Abteilen des ZNS durch die Synthese der Neurotransmitter zu bilden. Das kann zur Entwicklung der geistigen und der Verhaltensfähigkeiten beitragen. Autologe MSZ haben Vorrang gegenüber den Spender-MSZ. Als Ergebnis verläuft die Transplantation ohne Komplikationen und ohne Immunreaktionen. Als MSZ-Träger werden pharmazeutisch geeignete blutstillende Schwämme benutzt, welche sich später, nachdem sie ihre Funktion erfüllt haben, auflösen (Resorption).

Die Gehirnentwicklung wird auch durch die intravaskuläre (intravenöse oder intraarterielle) Injektion der autologen MSZ befördert. Eine solche Transplantation der MSZ ins Gehirn wird durch das Blutgefäßsystem des Gehirns möglich: Der Blutstrom überträgt die MSZ zum Schädigungsherd. Es wurde auch die Komplexeinführung der autologen MSZ erprobt, nämlich mittels intravaskulärer Injektion und mittels intrakortikaler Transplantation ins Hirn. In diesem Fall liegt eine intensivere Sättigung der Hirnrinde mit MSZ -Zellen vor. Der Schädigungsherd wird mit MSZ völlig versorgt.

## BESCHREIBUNG DER ERFINDUNG

Das Verfahren zur Gewinnung von Biotransplantat gemäß der Erfindung wird auf folgende Weise ausgeführt: Das Knochenmark des Menschen wird mittels Einstichs aus dem Brustbein (5-10 ml) oder aus der Darmbeinschaufel des Beckens (15-30 ml) im kleinen Operationsraum unter Regionalanästhesie entnommen. Das Punktat wird mit Heparin (500 Einheiten) vermischt und in einen Sterilbehälter mit einem luftdichten Deckel (Umfang 25 ml oder 50 ml) gesetzt. 10 ml Knochenmark werden bis auf 30 ml mit sterilem Nährboden DMEM (minimum Eagle media modified by Dulbecco) versetzt.

Diese Probe wird ins Prüfglas mit 20 ml von bis zur Raumtemperatur (18-23° C) aufgewärmtem Ficoll (Dichte 1,077 oder 1,115 g/l) gesetzt, ohne die Vermischung zuzulassen. Das Punktat wird auf Ficoll "aufgeschichtet" und im Laufe von 25 bis 60 Minuten bei einer Geschwindigkeit von 1000 - 2000 U/Min zentrifugiert. Danach wird die Zellfraktion an der Grenze von Ficoll und der Oberschicht des Überstands entnommen. Es wird dadurch sichtbar, dass an der Mediengrenze ein weißer Ring gebildet wird. Dieser Ring stellt die monozytäre Fraktion von Knochenmark dar. An der Oberfläche des Überstands verbleiben die Reststücke des Knochenmarks.

Die Zellsuspension wird durch die äquivalente oder zweifache Menge von DMEM-Medium verdünnt. Dieses Medium enthält Antibiotika (Penizillin 100 Einheiten/ml, Streptomyzin 100 $\mu$g/ml). Danach wird die Zellsuspension mittels Zentrifugierung im Laufe von 10 Minuten bei 1000 U/Min ausgefällt. Die Zellen werden im DMEM-Medium aufgeschwemmt, welches mit 20%igem Fötalochsenserum (FBS, fetal bovine serum) und Antibiotika (Penizillin 100 Einheiten/ml, Streptomyzin 100 $\mu$g/ml) ergänzt wird, und danach wieder mittels Zentrifugierung beim gleichen Regime ausgefällt. Der Zellniederschlag und die Oberschicht des Überstands werden ins Geschirr für die Züchtung übertragen und mit Züchtungsnährboden bedeckt, wobei dieser Nährboden DMEM unter Zusatz von 15-22 % FBS und Antibiotika enthält. Die Aussaat der Zellen wird mit einer Anfangsdichte von 75-200 Zellen pro cm2 vorgenommen und in den CO2-Inkubator für 18-52 Stunden gesetzt. Danach wird das Züchtungsmedium sorgfältig entnommen und die Zellkultur zweifach mit Hanks-Lösung gespült und mit frischem Züchtungsmedium bedeckt, welches bis +37° C aufgewärmt wird. Die Kultur wird in den CO2-Inkubator für 56-72 Stunden gesetzt. Das Kulturwachstum wird täglich beobachtet. Ggf. wird die Hälfte des Umfangs des konditionierten Züchtungsmediums durch den gleichen Umfang des frisch aufbereiteten Mediums ersetzt. Die Kultur wird unter Einsatz der Trypsinlösung auf das neue Geschirr mit einer Dichte von 1 - 2 x 103 Zellen pro 1 cm$^2$ überimpft. Die Kultur wird für 56-72 Stunden in den CO2-Inkubator gesetzt. Nach 2 bis 4 Passagen wird eine Zellpopulation erzeugt, welche durch eine hohe Homogenität nach den Positivmarkern (CD 90, CD 105, CD 106 und CD 44), d. h. den MSZ-Markern, gekennzeichnet ist. Die Ausbeute beträgt bis zu 99,9 % der gefärbten Zellen.

Während der Überimpfung und der Züchtung der Kultur wird die Menge der aus dem Glaskolben entnommenen Zellen mittels der Goryaev-Kammer gezählt. Dafür wird die Zellspülung aufgeschwemmt. Die Goryaev-Kammer wird mit der hergestellten Suspension gefüllt, und die Zellen werden in 25 großen Quadraten gezählt.

Die Auszählung der Zellen wird nach folgender Formel vorgenommen:

$$\sum Z \times 400 \times 1000 = \sum Z/ml,$$

dabei ist $\Sigma Z$ die Summe der Zellen in 25 Großquadraten, $\Sigma Z/ml$ ist die Zellmenge in 1 ml der Zellaufschwemmung. Vor dem Zählen werden die gezüchteten Zellen mit einer Hanks-Lösung und danach mit einer Trypsinlösung gespült. Anschließend wird der Glaskolben für 1-3 Minuten in den CO2-Inkubator mit visueller Überwachung der Zellaufrundung gesetzt. Bei einer Abrundung von mindestens 80 % der Zellen werden die Zellen vom Glaskolben mit dem warmen (t° = +37° C) Züchtungsmedium abgespült. Bei einer Glaskolbenfläche von 175 cm$^2$ beträgt der Umfang des benutzten Mediums 10 ml. Nach der Auszählung der Menge von Zellen, welche aus dem Glaskolben entnommen wurden, wird die folgende Passage vorgenommen. Die Qualität der MSZ wird anhand der Antikörper FITC, PE, biotin, Cy, mit Streptovidin ECD und mit Propidiumjodid (PI) beurteilt. Es wurden die Zellen mit Phänotyp CD 45-, CD 34-, CD 90+, CD 106+, CD 44+, CD 105+ bestimmt. Diese Zellen sind Mesenchymzellen. Die nicht mit Propidiumjodid eingefärbten Zellen sind lebensfähig.

Werden die Kennwerte bei der Durchführung der angemeldeten Abläufe (Vergrößerung oder Verminderung) geändert, so kann das genannte technische Ergebnis der Erfindung nicht mehr erreicht werden, weil dabei eine akute Abnahme der MSZ-Ausbeute entsteht und die Homogenität der Zellensuspension gestört wird.

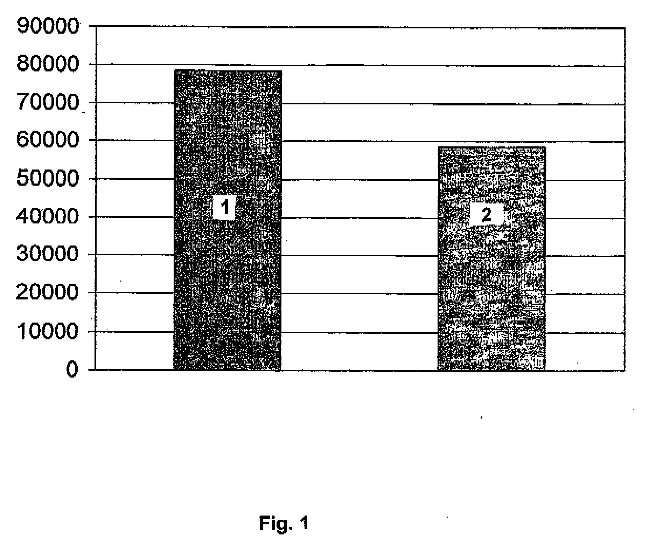

Die Zeit- und die Geschwindigkeitsreduzierung bei der Zentrifugierung verursacht den Verlust des Ringes aus mononuklearer Fraktion von Knochenmark. und baut damit die Gesamtausbeute des Produktes um 25-30 % ab. Das ist aus dem Diagramm in Fig. 1 ersichtlich: Die Y-Achse kennzeichnet die Menge von MSZ am 7. Tag der Zellenzüchtung. Kennwert 1 repräsentiert Zellen, welche aus dem mononuklearen Ring gewonnen wurden. Dieser Ring wird während der Zentrifugierung im Laufe von 30 Minuten bei 1000 U/Min gebildet. Der Kennwert 2 repräsentiert Zellen, welche aus der Masse gewonnen wurden, die keinen deutlichen mononuklearen Ring darstellen. D. h., dies kommt beim Verlust des Ringes zustande, wenn das Saatgut mittels Zentrifugierung im Laufe von 20 Minuten bei 500 U/Min abgeleitet wird.

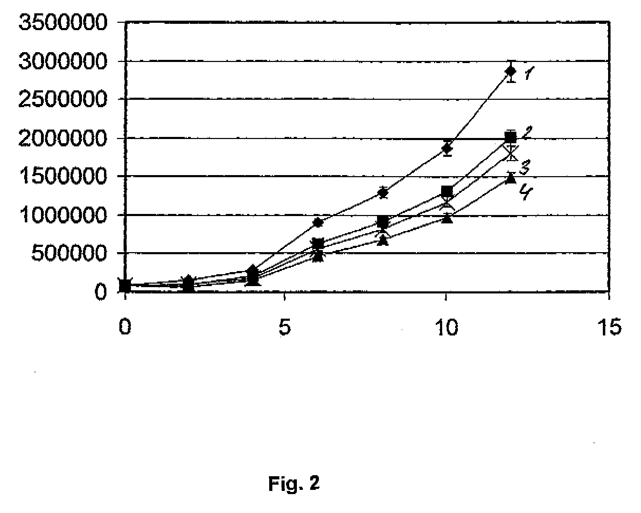

Wenn die Serummenge im Züchtungsmedium der MSZ verringert oder das Nährmedium geändert (z. B. Einsatz von RPMI) wird, so wird die Dauer der Vermehrung des Zellmaterials bis zu 3 Monaten verlängert. Die Angaben sind in Fig. 2 dargestellt. Die Y-Achse gibt die Zellenmenge an, und die X-Achse kennzeichnet die Vermehrungstage. MMSZ - MSZ des Menschen.

Dieses Diagramm stellt die Ergebnisse der Wachstumsanalyse der Zellkulturen von MSZ bei verschiedenen Bedingungen dar.

1. MMSZ ist die Norm: gemittelte Angaben, welche beim Einsatz der Methode nach dem Patent ermittelt wurden.
2. MMSZ - 10 % des Serums, gemittelte Angaben, welche beim Einsatz von 10 % des Fötalserums ermittelt wurden.
3. MMSZ - 5 % des Serums, gemittelte Angaben, welche beim Einsatz von 5 % des Fötalserums ermittelt wurden.
4. MMSZ - RPMI, gemittelte Angaben, welche beim Einsatz von RPMI-Medium ermittelt wurden.

Aus dem Diagramm ist ersichtlich, dass diese Methode optimal ist und dass die Unterschiede glaubwürdig sind.

Der Ficolltemperaturabbau bis zu +10° C verursacht einen Gradientenverlust, und die Temperaturerhöhung steigert die toxischen Eigenschaften von Ficoll.

Beim Vergleich des erfindungsgemäßen Verfahrens mit den bereits bekannten Methoden, bei denen die Zellenableitung auch mittels Zentrifugierung erfolgte, sei Folgendes bemerkt: Die dem Phänotyp nach homogene Zellpopulation der MSZ gemäß dieser Erfindung wird praktisch sofort gewonnen, indem die Suspension des heparinisierten Punktats von Knochenmark im DMEM anschließend im Ficoll zentrifugiert wird. Bereits während der Ableitung der Zellen an der Mediengrenze DMEM - Ficoll enthält das "Häutchen" die homogenen Zellen MSZ, welche durch sechs Marker gekennzeichnet sind: CD 45-, CD 34-, CD 90+, CD106+, CD 44+, CD105+. Somit unterscheidet sich die Zellpopulation MSZ nach der angemeldeten Erfindung vom bekannten Verfahren durch umfangreichere und genauere Differenzierung, nämlich durch das Vorhandensein vom CD106+ - Marker, welcher eine zusätzliche Charakteristik für Homogenität der MSZ darstellt.

Die Bearbeitung von Geschirr ist gemäß der Erfindung nicht mehr notwendig. Das benutzte Medium ist zugänglich, genormt, weit und breit anwendbar und stellt kein spezielles Medium dar. Die Aussaatdichte der MSZ beträgt 75-200 Zellen pro cm$^2$, und die Inkubationsdauer beträgt 56-72 Stunden. Somit wird das Endprodukt gemäß der Erfindung wesentlich günstiger und weist eine hohe Ausbeute und Homogenität auf.

Die Urheber der Erfindung arbeiteten mit MSZ-Zellen des Rezipienten (des Menschen), welche aus Knochenmark abgeleitet wurden. Sie behandelten die Zellen mit keinen chemischen Reagenzien, um den negativen Einfluss auf die Zellen zu minimieren.

Infolge der Verwendung der aus Knochenmark des Patienten entnommenen Zellen wurden als Saatgut die für die Biotransplantation erforderlichen Zellen gewonnen, deren Menge ihren Gehalt im Knochenmark um einige Dutzend Male überschreitet. Nach der Subkultivierung und Entnahme der Glaskolben wurde ein Konzentrat mit folgendem Gehalt erhalten: 8-10 x $10^6$ MSZ in 1 ml.

Das Biotransplantat zum therapeutischen Einsatz wird in Form von einer Zellsuspension in 10%igem autologem Blutserum auf der Grundlage der physiologischen Lösung gewonnen. Die Zellsuspension enthält lebensfähige autologe Mesenchymstammzellen (MSZ), die nach Zellmarkern CD 44+, CD 90+, CD 105+, CD 106+, CD 45-, CD 34- homogen sind.

Anstatt eines 10 %igen autologen Blutserums auf der Grundlage der physiologischen Lösung (aufgeschwemmtes Lösemittel) können auch andere Lösemittel benutzt werden. Sie werden aus pharmazeutisch geeigneten Mitteln gewählt: Autoplasma in der physiologischen Kochsalzlösung, Autoplasma, Autoserum mit beliebigem %-Anteil von 5 bis 100 sowie flüssige Glukose 10 %, 5 %, 7 % auf der Basis der physiologischen Kochsalzlösung. In 1 ml des Biotransplantats sind 0,7 - 1,2 x $10^6$ MSZ enthalten.

Die Vorzugsvariante der Zusammensetzung zur Neurotransplantation: Autoserum auf der Basis der physiologischen Kochsalzlösung.

Autologe Zellen im Gehirn des Rezipienten verursachen keine Komplikationen in Bezug auf Gewebeverträglichkeit. Sie sind physiologisch verträglich und stellen einen positiven Effekt bei der Behandlung sicher.

Die erste unspezifische Wirkung (mit Dauer bis zu 6 Monaten) ist dadurch bedingt, dass die verpflanzten MSZ im Laufe des starken Wachstums den ganzen Bestand an ihren neurotrophischen Faktoren, unterschiedlichen Aminosäuren und Zytokinen aktiv ableiten. Der Sekundäreffekt hängt damit zusammen, dass die eingeheilten Zellen, dank ihrem Charakter, sich in das neuronale und funktionelle Hirnsystem des Rezipienten integrieren, indem sie damit synaptische Verbindungen bilden. Sie üben eine normalisierende Wirkung nicht nur auf die pathologisch funktionierenden Strukturen sondern auch auf die gesamte Hirnaktivität aus. Der positive Effekt dieser Abläufe kann möglicherweise erst im Laufe einiger Jahre spürbar werden.

Das Biotransplantat kann bei der Behandlung von Schädigungen der intellektuellen und mnestischen Funktionen mit unterschiedlicher Ätiologie eingesetzt werden. Diese große Gruppe von Störungen umfasst die ausgeprägten Formen der Retardation.

Die Retardation bei Kindern wird in allen Ländern festgestellt. Laut Statistik haben 0,25 - 0,50 % der Bevölkerung einen Intelligenzkoeffizienten (IQ) unter 50, was eine schwere und ausgeprägte Retardation kennzeichnet. Die Prognosen für den Verlauf sind ungünstig. Der angeborene Schwachsinn bei Menschen wird durch zwei obligatorische Merkmale charakterisiert: 1) allgemeine psychische Unterentwicklung mit vorwiegend geistiger Insuffizienz; 2) Abwesenheit von Progression, welche den pathologischen Vorgang anzeigt (G.E. Sukhareva, Vorlesungen über Psychologie des Kinderalters. M. Medizin. 1974 - 320 S.).

Eine der Besonderheiten von Pathogenese der Oligophrenie besteht darin, dass die Missbildung (Dysontogenie) infolge der Schädigung des Organismus entsteht, welcher seine Entwicklung noch nicht abgeschlossen hat. Darin besteht sein grundsätzlicher Unterschied zu einem Fehler infolge einer Störung im bereits geformten Organismus.

Das klinische Bild der Dysontogenien, darunter der angeborenen Anomalien der psychischen Entwicklung, unterscheidet sich wesentlich von der Klinik der Schädigungen der vollständig gebildeten (entwickelten) Organe und Systeme. Im ersten Fall liegt eine Entwicklungsstörung vor, und im zweiten Fall handelt es sich um einen erworbenen Fehler der vorher normalen Funktionen. Die Hauptbesonderheit der diffusen "totalen" Unterentwicklung ist die allgemeine Gemütsstörung.

Es wurde bemerkt, dass bei den Kranken Merkmale der Unterentwicklung von Intelligenz, Denken, anderen psychischen Funktionen (Wahrnehmung, Gedächtnis, Aufmerksamkeit, Sprechen, Motorik, Emotions- und Willenssphäre usw.) feststellbar sind.

Das Schädigungsbild hängt nicht nur von der Natur und der Intensität der Schädigungseinwirkung ab, sondern vor allem von den Besonderheiten jener Periode der Ontogenese, bei der das sich entwickelnde Organ und der gesamte Organismus betroffen worden ist, d.h. das Schädigungsbild ist vom chronogenen Faktor abhängig. Je nach Tiefe der psychischen Unterentwicklung wird zwischen 3 Retardationsstufen unterschieden: Idiotie (IQ < 20), Imbezillität (IQ = 20 - 50), Debilität (IQ = 50 - 70).

Zur Neurotransplantation werden eigene Mesenchymstammzellen eingesetzt, welche aus dem Knochenmark des Kranken hergestellt und des Weiteren in vitro kultiviert werden. Die Zellen werden in die Schädigungsbereiche, d.h. parenchymatös in Form des oben genannten Biotransplantats eingeführt. Die Zellen zur Behandlung werden auch intravaskulär eingeführt: i/a - intraarteriell, i/v - intravenös. Dabei beträgt die Gesamtmenge der eingeführten MSZ 7 bis 25 Mio.

Um die Neurotransplantation durchzuführen, wird Biotransplantat benutzt, welches 40-45 Mio. junge MSZ mit einer Konzentration von 0,7 - 1,2 x $10^6$ in 1 ml des Biotransplantats enthält. Es wird der modifizierte Stent eingeführt, der der Träger der Zellen ist. Als Träger werden vorwiegend blutstillende Schwämme eingesetzt. Pro einem genormten hämostatischen Schwamm 9 x 9 werden normalerweise 20 ml der Zellsuspension verwendet. Die Fragmente des mit MSZ gesättigten Stents mit einer MSZ-Menge von 7 - 12 x $10^6$ pro Zone werden in einen oder mehrere im voraus bestimmte Bereiche der Hirnrinde verpflanzt. Dazu zählen vor allem der Bereich der funktionellen Brodmann-Zonen 44, 43, 46, 24, 25, 22, 41 und 42. Ferner wird der Stent dem allmählichen Auflösen ausgesetzt. Die darin enthaltenen MSZ werden mit dem Blutstrom ausgewaschen und integrieren ins Hirnparenchym. Um einen größeren Effekt zu erreichen, werden in manchen Fällen gleichzeitig intraparenchymatöse und intravaskuläre MSZ-Einführungsverfahren angewendet, d. h. während der Operation werden die beiden Zelleinführungsverfahren vereinigt. Dabei erfolgt eine zusätzliche Einführung mit der Dosierung von 0,5 - 0,7 x $10^6$ der autologen Mesenchymstammzellen pro 1 kg des Patientengewichts.

Die Speicherung der autologen MSZ für ihren Einsatz als Biotransplantat verläuft in der Zeit. Deswegen können solche Situationen entstehen, dass die Speicherung (die Produktion oder Ansammlung) der Zellen abgeschlossen ist, während der Patient seinem Befinden nach zur Behandlung noch nicht bereit ist. In solchen Fällen wird das Zellmaterial der Kryokonservierung ausgesetzt. Die Kryokonservierung erhält die MSZ unverändert. Nach der Auftauung verfügen die Zellen über alle ihre Eigenschaften, weisen die gleichen Charakteristiken ihrem Phänotyp nach auf und bleiben lebendig. Die Kryokonservierung wurde unter Einsatz von Dimethylsulfoxid als Kryoschutzmittel vorgenommen, weil Dimethylsulfoxid pharmazeutisch geeignet und mit verwendeten Lösemitteln verträglich ist. Die Lösemittel werden in der Suspension bei der Behandlung von autologen Zellen eingeführt.

Nach der Kryokonservierung wurden die Biotransplantate bei der Behandlung von Patienten genau wie die oben beschriebenen frischen Zellen eingeführt. Die Behandlungsergebnisse sind mit der Anwendung der frisch zubereiteten Biotransplantate vergleichbar.

Bei ausgeprägten anatomischen Änderungen (oder wenn sie nicht vorliegen) der funktionell bedeutsamen Hirnzonen werden die MSZ nur intravaskulär (i/a - mit einer Geschwindigkeit von 2 ml/Min oder i/v mit einer Geschwindigkeit von 6 ml/Min) eingeführt. Dabei beträgt die Gesamtmenge der eingeführten MSZ 7 bis 25 Mio. Die Menge der MSZ wird berechnet unter Annahme von 0,5 - 0,7 x $10^6$ Zellen pro 1 kg des Patientengewichts. Für die Transplantation wurden entweder frisch aufbereitete Zellen angewendet, wenn die Behandlung unter Einsatz von diesen Zellen vorgenommen wurde, oder kryokopierte MSZ, wenn sie in der Behandlung verwendet wurden.

Eine wichtige Bedingung ist die Verkürzung des Zeitraums zwischen der Entnahme der MSZ aus kultiviertem Medium und ihrer Transplantation unmittelbar ins Gehirn des Kranken, da in jeder Minute dieser Zeit eine bestimmte Menge der Zellen lebensunfähig wird, was das Endergebnis negativ beeinflusst.

Das Verfahren gemäß der Erfindung wurde durch NIPNI (Bechterevs Wissenschaftliches Forschungsinstitut für Psychoneurologie) Abteilung für Chirurgie der Nerven- und psychische Krankheiten erprobt.

Das Biotransplantat kann ebenfalls bei der Behandlung von pathologisch geänderten Gehirngeweben benutzt werden, die auf Folgeerscheinungen eines Schädelhirntraumas (SHT) oder der akuten Störung des Gehirnkreislaufs (ASGK) zurückzuführen sind.

Folgeerscheinungen des SHT ist eine evolutionsmäßig vorausbestimmte und genetisch festgelegte Gesamtheit von Vorgängen als Reaktion auf die Schädigung des Gehirns. Die Folgeerscheinungen umfassen auch ständige Störungen der anatomischen Ganzheit des Gehirns, der Hüllen und der Schädelknochen, welche infolge von akutem SHT entstehen und in der mittel- und langfristigen Zeitperiode erhalten bleiben.

Bei 70 % der Betroffenen mit leichtem und mittlerem Schweregrad von SHT werden in einem bestimmten entfernten Zeitraum verschiedene Erscheinungen der zerebralen Pathologie erkannt. Bei 30 % der Betroffenen wird eine wesentliche Minderung der Erwerbsfähigkeit bis zur kompletten Behinderung festgestellt.

Nach dem SHT entwickelt sich ·eine Gesamtheit von reparativen und dystrophischen Körperreaktionen, deswegen sind Folgeerscheinungen nach jedem SHT praktisch unvermeidlich. Im klinischen Sinne wird jedoch über Folgeerscheinungen nur dann gesprochen, wenn sich infolge der Schädigungen (Besonderheiten der Reaktionsfähigkeit des Gehirns und des gesamten Organismus, altersbedingte Faktoren usw.) ein ständiger pathologischer Zustand entwickelt, der behandelt werden muss.

Zurzeit werden folgende Gruppen von Folgeerscheinungen der SHT unterschieden:

- gewebebedingte (Hirn- und Schädel-) Folgeerscheinungen: Hirnatrophie, Arachnoiditis, Hüll-Hirnnarben, Schädigung der Hirnnerven, Schädelfehler, Fremdkörper usw.;
- liquordynamische Folgeerscheinungen: Hydrozephalus, Porenzephalie, Liquorfistel, Liquorzysten usw.
- vaskuläre Folgeerscheinungen: ischämische Schädigung, chronische Hämatome, Aneurysmen, arteriosinus Anastomose, Sinusthrombose usw.
- vermischte Folgeerscheinungen.

Die Gehirnkreislaufsstörungen (Insulte) entstehen infolge OHMK und verursachen eine komplette Behinderung. In der RF gibt es über 400.000 Insulte.

Während der Behandlung müssen die Körperfunktionen wiederhergestellt sowie die Fehler beseitigt werden, die durch den bereits endgültig geformten Organismus erworben worden sind, wenn dieser Organismus vor SHT oder OHMK eine normale Funktion und Entwicklung, Fertigkeiten und Intelligenz besessen hat.

Die Änderungen betreffen Intelligenz, Denken, Gedächtnis, Sprechen, Motorik, Emotions- und Willenssphäre, Wahrnehmung usw. Dabei entwickeln sich die posttraumatischen Psychosen. Die Störungen im Körper hängen von der Tiefe und der Lokalisation der Schädigung von Gehirn und Nervengewebe ab.

Das Knochenmark wird dem Patienten entnommen, um die autologen MSZ mittels Subkultivierung der Zellmasse zu speichern. Es wird auch das Autoblut entnommen, um das Biotransplantat zu bekommen. Das Biotransplantat wird in den Operationsraum weitergeleitet. Das Biotransplantat stellt autologe MSZ dar, welche sich in dem Autoblutserum befinden. Das Zellmaterial wird ausgehend von 5 Mio. in 1,0 ml des Autoserums vorbereitet. Um die Infizierungsmöglichkeit beim Öffnen der Behälter mit Zellen auszuschließen, werden die Zellen zusätzlich mit Antiseptika behandelt. Vor der Einführung ins Gehirn werden sie auf 34 - 36° C aufgewärmt und dann auf den Träger (blutstillenden Schwamm) übertragen.

Die intrazerebrale Einführung der autologen MSZ wird folgenderweise vorgenommen: Der Zugang an die pathologisch geänderten Gewebe und ihre chirurgische Bearbeitung erfolgt nach genormten Methoden. Es wird eine sorgfältige Blutstillung vorgenommen.

Der Bereich der Hirnrinde zur Durchführung der Neurotransplantation (mit größten morphologischen und funktionellen Störungen) wird im voraus vor der Operation bestimmt. Dabei wird die MSZ-Einführung unmittelbar in Bindegewebenarbenbildungen und in die zystischen Höhlungen vermieden. Die Transplantation wird in die perifokalen Gehirnzonen mit ausreichender Blutversorgung vorgenommen. Es handelt sich dabei zu allererst um das Gebiet der funktionellen Brodmann-Zonen 44, 43, 46, 24, 25, 22, 41, 42. Bei Vorhandensein von Änderungen in den funktionsmäßig bedeutsamen Zonen (Brok-Zone, Vernike-Zone, Zentralwirkungszone, Hinterhauptlappenrinde) erfolgt der Zugang an diese Zonen außerhalb der Projektion, um der Steigerung des neurologischen Defizits in der Nachoperationsperiode vorzubeugen.

Der Träger mit Zellmaterial wird in die perifokale Zone subkortikal verpflanzt. Danach werden jegliche Manipulationen an der Hirnwunde vermieden. Der Gesamtumfang des Zellmaterials beträgt 20-25 Mio. Zellen. Die feste Hirnhaut wird dicht ohne Dränage des Subduralraums zugenäht. Die Wunde wird schichtweise mit Erhaltung der subkutanen Dränage eingenäht.

Des Weiteren löst sich der Träger (modifizierter Stent) allmählich auf. Die darin enthaltenen MSZ werden mit dem Blutstrom ausgewaschen und dringen in das Hirnparenchym ein.

Bei der Stenteinführung in den subkortikalen Kern werden die MSZ in 5 ml des Lösemittels (Umfang der Zellsuspension) eingeführt.

Vor der Einführung der MSZ wird eine chirurgische Bearbeitung von pathologischen Bildungen nach genormten Methoden vorgenommen. Das ist erforderlich, weil die Einheilungsfähigkeit und die funktionelle Aktivität der verpflanzten Zellen von der Sauerstoffversorgung und der Versorgung mit Nährstoffen wesentlich abhängen. Werden notwendige Bedingungen eingehalten, so ist die Neoangiogenese im Gewebe zur Ausprägung seiner funktionellen Besonderheiten möglich.

Wenn nach SHT ein Hämatom vorliegt, so wird das Hämatom mittels chirurgischen Eingriffs entfernt. Danach wird im Traumabereich der hämostatische Schwamm gesetzt, welcher anschließend mit einer Suspension der autologen MSZ bei einer Konzentration von 2 Mio. Zellen pro 1 $cm^3$ des Schwamms benetzt wird (die Suspension wird auf den Schwamm aufgetragen). Die Zellen werden bei der Behandlung nur einmal benutzt.

Die intrazerebrale Einführung von MSZ erfolgt auf einem Träger. Bei der vorgeschlagenen Ausführung handelt es sich um einen hämostatischen Schwamm. Die Menge der in eine Zone der Hirnrinde eingeführten MSZ beträgt 7 bis 12 Mio. Die Anzahl der Zonen variiert.

Die Einführung der autologen phänotypmäßig homogenen MSZ erfolgt intravaskulär, nämlich intravenös oder intraarteriell. Die Geschwindigkeit bei intravenöser Einführung beträgt 2 ml/Min. und bei intraarterieller Einführung 6 ml/Min. Die Menge der MSZ wird ausgehend von 0,5 - 0,7 Mio. Zellen pro 1 kg des Patientengewichts berechnet.

Es wurde eine Methode mit Einführung der MSZ mittels gleichzeitiger oder konsequenter intravaskulärer und intrazerebraler Einführung erprobt. Das Behandlungsergebnis war positiv. Die intravaskuläre Einführung erfolgte mittels Biotransplantat, welches aus MSZ im 10%igen autologen Blutserum auf der Basis der physiologischen Lösung bei einer Zellenkonzentration von 0,7 - 1,2 x $10^6$ in 1 ml Transplantat bestand. Die intravaskuläre Einführung erfolgte ausgehend von bis zu 1,2 Mio. MSZ pro 1 kg des Patientengewichts.

Die Einführung von MSZ ins Gehirn erfolgte intraparenchymatös ausgehend von 7-12 Mio. Zellen pro Zone.

Der klinische Effekt wurde durch Angaben von paraklinischen Forschungen (EEG, US-Blutflussuntersuchung, PET, psychologische Untersuchungen u.a.m.) bestätigt. Der Behandlung wurden 20 Kranke unterzogen. Ein positiver Effekt wurde bei allen Kranken festgestellt.

**BEISPIELE FÜR AUSFÜHRUNG DER ERFINDUNG**

Beispiel 1: MSZ des Menschen

10 ml Knochenmark wurden aus dem Brustbein unter Lokalanästhesie in eine sterile Spritze aufgenommen, die 500 Einheiten von Heparin enthielt.

Für die weitere Arbeit wurde das Punktat in einen Sterilbehälter übertragen. Der Sterilbehälter stellt das Prüfglas mit einem Umfang von 50 ml und mit luftdichtem Deckel dar. Der Vorgang für die Fraktionierung von Knochenmark (Phänotypisierung von Knochenmark) erfolgte in Sterilräumen, nämlich im Laminarschrank nach der 2. Schutzklasse, Kajar.

Das erhaltene Punktat wurde mit sterilem Nährboden DMEM ergänzt, indem der Probenumfang bis auf 30 ml gebracht wurde.

20 ml Ficoll (Dichte 1,077 g/l) wurden in das sterile 50 ml große Prüfglas gesetzt, ohne die Vermischung der Medien zuzulassen. 30 ml Punktat wurden oberhalb des Ficolls aufgeschichtet. Dieses Prüfglas wurde in die Zentrifuge mit "Bucket-Rotor" gesetzt. Das Punktat wurde 40 Minuten lang bei einer Geschwindigkeit von 1500 U/Min und bei einer Temperatur von t° = 20 - 22° C (Raumtemperatur) zentrifugiert. Die Zellen an der Mediengrenze (es wurde ein spezifischer weißer Ring gebildet, welcher die monozytäre Fraktion von Knochenmark darstellte) wurden ausgewählt. Die an der Überstandsoberfläche gebliebenen Knochenmarkteilchen wurden gesammelt.

Das gesammelte Material wurde ins sterile Prüfglas gesetzt und mit Nährboden DMEM ergänzt, dessen Umfang den Umfang des entnommenen Materials um das Zweifache überschritt.

Die resultierende Suspension wurde im Laufe von 5 Minuten bei einer Geschwindigkeit von 1500 U/Min und bei Raumtemperatur zentrifugiert.

Der Überstand wurde entfernt. Der Niederschlag wurde mit 12 ml des Züchtungsmediums ergänzt, welches aus DMEM (80 %) und aus Fetalserum von Kühen (20 %) bestand.

Die Zellsuspension wurde auf den Glaskolben mit einer Fläche von 75 cm$^2$ zwecks Züchtung übertragen. Der Glaskolben wurde in den $CO_2$-Inkubator für 48 Stunden gesetzt. Nach der Explantation aus dem Glaskolben wurde das Züchtungsmedium mit den in der Suspension übriggebliebenen Zellen entnommen. Der Glaskolben wurde mit einer Hanks-Lösung, 10 ml, gespült. Die Spüleffektivität wurde unter dem Inversionsmikroskop je nach der Menge der in der Suspension übriggebliebenen Zellen (hämopoetische Zellen) eingeschätzt. Das bis zu +37° C erwärmte Züchtungsmedium (80 % DMEM, 20 % Fetalserum von Kühen), Umfang 12 ml, wurde in den Glaskolben für die Züchtung zugegeben. Danach wurde der Glaskolben für 3 Tage in den CO2-Inkubator gesetzt. Das Kulturwachstum wurde täglich beobachtet, dabei wurde die Kultur ernährt. Dafür wurden 6 ml des konditionierten Mediums durch 6 ml des frisch aufbereiteten Mediums ersetzt.

Am 3. Tag der Züchtung wurde der Projektionsüberzug des Glaskolbens mit den Zellen der primären Knochenmarkkultur anhand eines Inversionsmikroskops eingeschätzt. Dieser Überzug betrug über 80 %. Deswegen wurde die Kultur in den Glaskolben mit einer größeren Fläche, nämlich 175 cm$^2$, überimpft. Passage 1 (P1 ).

Folgende Prozeduren wurden für die Überimpfung vorgenommen:

1.   Entfernung des konditionierten Züchtungsmediums;
2.   Spülung der gezüchteten Zellen mit 10 ml Hanks-Lösung;
3.   Spülung der gezüchteten Zellen mit 4 ml Trypsin-Lösung;
4.   Wiederholte Spülung mit Trypsin, 4 ml;
5.   2 Minuten lange Behandlung des Glaskolbens im CO2-Inkubator (Zellenaufrundungsdauer; der Vorgang wurde jede Minute überwacht); innerhalb dieser 2 Minuten erfolgte die Aufrundung von über 80 % Zellen;
6.   Abspülung der Zellen vom Glaskolben, 10 ml, mit dem bis zu +37° C erwärmten Züchtungsmedium;
7.   Einsetzen der Zellsuspension ins sterile Prüfglas mit einem Umfang von 15 ml;
8.   Kontrolle der Qualität der Zellenentnahme aus dem Glaskolben anhand des Inversionsmikroskops: Auf dem Glaskolben bleiben einzelne Zellen übrig;
9.   Auszählung der Zellen unter Anwendung der Goryaev-Kammer. Die Zellenmenge betrug 8,4 Mio;
10.   Die Zellsuspension wurde in den sterilen Glaskolben für die Züchtung mit einer Gesamtfläche von 175 cm$^2$ übertragen;
11.   15 ml des Züchtungsmediums wurden in den Glaskolben hinzugefügt, und die Zellsuspension wurde mittels drehender Bewegungen vermischt;
12.   Der Glaskolben wurde in den $CO_2$-Inkubator gesetzt.

Am 2. Tag der Züchtung betrug der Projektionsüberzug des Glaskolbens nach P1 über 80 % der Gesamtfläche von 175 cm$^2$ (die Beurteilung erfolgte anhand des Inversionsmikroskops). Die Kultur wurde in drei Glaskolben mit einer Fläche von jeweils 175 cm$^2$ überimpft, Passage 2 (P2).

Der Vorgang der Zellzüchtung in jedem Glaskolben wurde in Übereinstimmung mit der Beschreibung für Passage 1 unter PP. 1-9 wiederholt.

Die Auszählung der Zellen anhand der Goryaev-Kammer beim Schritt 9 ergab 15,6 Mio. Zellen.

Beim Schritt 10 wurde der Umfang der Zellsuspension mit Zugabe von Züchtungsmedium bis auf 15 ml gebracht. Danach wurden jeweils 5 ml der Zellsuspension auf drei sterile Glaskolben mit einer Fläche von je 175 cm$^2$ übertragen. Jeder Glaskolben wurde mit 20 ml Züchtungsmedium ergänzt und in den CO2-Inkubator gesetzt. Die Zellen wurden 4 Tage lang bis zum Projektionsüberzug des Glaskolbens um mehr als 80 % gezüchtet. Die Zellkultur wurde am 3. Tag ernährt. Dafür wurden 12 ml des konditionierten Züchtungsmediums durch 12 ml des frisch aufbereiteten Mediums ersetzt.

Nach der Passage 2 (P2) stieg die Zellenausbeute auf 58,3 Mio. Zellen.

Passage 3 (P3) erfolgte ähnlich wie P2. Dabei wurden die Kulturen von jedem Glaskolben in 3 neue Glaskolben mit einer Fläche je 175 cm$^2$ überimpft.

Nach P3 wurde die Zellkultur mittels Antikörperfärbung gegen die Oberflächenantigene anhand des Durchlauf-Zytoströmungsmessers (Beckmann Coulter) immunphänotypisiert. Dabei wurde indirekte Fluoreszenz benutzt. Die Zellen entsprechen dem Immunophänotyp der MSZ, weil sie nach den Markern CD 45-, CD 34-, CD 90+, CD 106+, CD 44+, CD 105+ positiv sind. Die Zellen sind lebensfähig, weil sie mit Propidiumjodid nicht gefärbt sind. Die Ergebnisse der Analyse sind in Tabelle Nr. 1 enthalten.

**Tabelle Nr. 1**

| Charakteristik der nach Passagen resultierenden Mesenchymstammzellen | | | | | | |
|---|---|---|---|---|---|---|
| Prüfglas-Nummer | Antikörper FITC | Antikörper PE | Antikörper Biotin | Antikörper Cy | Streptavidin ECD | PI |
| 1 | - | | | | + | -- |
| 2 | CD 44 | CD 105 | CD 106 | CD 90 | + | -- |
| 3 | CD 71 | | | | -- | -- |
| 4 | CD45 | CD 34 | | | -- | + |
| 5 | | CD 106 | | | -- | -- |

Die Zellenausbeute betrug nach der 3. Passage (P3) 159 Mio. Zellen. Sie zeichneten sich durch eine hohe Homogenität von 99 % aus. Alle Zellen waren lebensfähig.

Beispiel 2: Verfahren zur Einschätzung der Qualität von MSZ.

1-1,2 Mio. von Zellen im Umfang von 250 µl werden unter sterilen Bedingungen entnommen. Die Suspension wird im Prüfglas mit der automatischen Pipette umgerührt. Die Suspension wird portionsweise zu je 50 µl in die Prüfgläser übertragen. Die Prüfgläser werden mit Ziffern von 1 bis 5 markiert. Mittels der automatischen Pipette werden Antikörper gemäß Tabelle 2 in die Prüfgläser eingebracht:

**Tabelle Nr. 2**

| Phänotypisierung von MSZ | | |
|---|---|---|
| Prüfglas-Nummer | Antikörper FITC | Antikörper PE |
| 1 | Isotyp Kontrolle und CD 45 | |
| 2 | CD 45 | CD 34 |
| 3 | CD 71 | CD 34 |
| 4 | CD 45 | CD 90 |
| 5 | CD 45 | CD 106 |

Die Antikörper werden portionsweise à 10 µl in Übereinstimmung mit ihrer Gebrauchsanleitung zugegeben. Der Inhalt der Prüfgläser wird mittels Pipettierens vermengt und innerhalb von 25 Minuten bei t° = 20 - 22° C (Raumtemperatur) im Dunkelraum, ohne Lichtzugang, inkubiert. Jedem Prüfglas wird je 1 µl Hanks-Lösung zugegeben und bei einer Beschleunigung von 400 U/Min im Laufe von 5 Minuten zentrifugiert. Der Überstand wird aus den Prüfgläsern entfernt. Die Prüfgläser Nr. 1 und Nr. 2 werden mit 2,5 ml Streptavidin ECD versehen, vermengt und, wie oben angeführt, 15 Minuten lang inkubiert. Danach wird 1 ml Hanks-Lösung zugegeben, wieder im Laufe von 5 Minuten bei einer Beschleunigung von 400 U/Min zentrifugiert, und der Überstand wird entfernt. Die Zellsuspension wird in 400 $\mu$l der Hanks-Lösung wieder aufgeschwemmt. Die erzeugte Zellaufschwemmung wird mittels Pipettierens vermengt.

0,5 $\mu$l Propidiumjodid werden ins Prüfglas Nr. 4 eingebracht, und wie im Vortext beschrieben, vermengt. Danach wird das Prüfglas 5 Minuten lang in Dunkelheit bei Raumtemperatur inkubiert. Die gefärbte Zellsuspension wird im Durchfluss-Zytoströmungsmesser EPICS XL (Beckmann, Coulter, USA) oder in einem ähnlichen Durchfluss-Zytoströmungsmesser analysiert.

Die für die Ableitung der MSZ aus Knochenmark verwendeten Verfahren können zur Ableitung der MSZ aus Knochenmark von Säugetieren eingesetzt werden, wenn sie für die Tierheilkunde oder für die Forschungsarbeit zu verschiedenen Zwecken erforderlich sind.

Beispiel 3: MSZ der Tiere

Das Knochenmark wurde weißen Ratten entnommen. Die Suspension wurde aus den Oberschenkelknochen der Ratten sofort nach der Dekapitation abgeleitet.

Das Knochenmark wurde folgenden Prozessen, wie im Beispiel 1 beschrieben, unterzogen: Disaggregation durch Heparin; Aufbereitung seiner Suspension im DMEM-Medium; danach wurde die resultierende Suspension sorgfältig und ohne Vermengung auf Ficoll aufgeschichtet; Zentrifugierung der Suspension erfolgte im Ficoll mit einer Dichte von 1115 g/l. Zentrifugierungsdauer - 40 Minuten, Umdrehungszahl - 1500 U/Min, Ficoll-Temperatur +20° C. An der Mediengrenze wurde ein Ring, das sogenannte "Häutchen" gebildet, welches aus Knochenmarkzellen bestand. Die Zellsuspension wurde im gleichen Umfang mit DMEM-Medium verdünnt. Danach wurde die Zentrifugierung wiederholt. Die gewonnenen MSZ-Zellen waren zu 98 % nach dem Phänotyp CD 45-, CD 90+ homogen (die Zellen wurden nach 2 Markern geprüft).

Die Zellen wurden ins genormte Geschirr für die Züchtung gesetzt. Als Züchtungsmedium wurde DMEM unter Zusatz von 20 % FBS eingesetzt. Es wurden 3 Subkultivierungen durchgeführt. Von 1 ml Knochenmark wurden 1,55 $\times$ $10^6$ Zellen produziert. Laut Reaktion mit Propidiumjodid (Einfärbbarkeit) waren die Zellen lebensfähig. Die Homogenität der Zellen nach dem Phänotyp betrug 90,5 % (Marker CD 45-, CD 90+, CD 44+, CD 34-, CD 105+, CD 106+).

Beispiel 4 (nicht im Rahmen der Erfindung):

Patientin K., geb. 1981. Der Schwangerschaftsverlauf bei der Mutter war von Toxikose begleitet. Die Geburt erfolgte mit ausgeprägter Asphyxie. Die ersten 7 Lebenstage befand sich die Patientin (das Kind) in der Reanimationsabteilung. Die Entwicklung in der Frühzeit erfolgte mit Retardation. Das Kind konnte sich erst nach 1,5 Jahren setzen, und selbstständiges Gehen war erst mit 5 Jahren möglich. Retardation der psychischen und Sprechentwicklung bestand von früher Kindheit an. Die Kranke wurde ständig mit unterschiedlichen Medikamenten ambulant und mehr als 12 Mal in verschiedenen Krankenhäusern behandelt. Sie war lernunfähig. Es wurde keine positive Dynamik im Zustand der Kranken festgestellt.

Die Krankenhauseinlieferung erfolgte im Alter von 24 Jahren. Es liegt praktisch kein produktiver Kontakt mit der Kranken vor. Die Kranke kann sich so gut wie überhaupt nicht behelfen. Beim Ansprechen versteht sie die Rede nur teilweise. Die Anzahl richtig gebrauchter Wörter beträgt max. 3 bis 8. Max. 5 bis 7 Befehle werden richtig ausgeführt. Unartikuliertes Sprechen. IQ-Niveau entspricht der Retardation in der Idiotie-Stufe (< 20). Das Syndrom der motorischen Hyperaktivität mit zerstörerischer Neigung ist ausgeprägt. Die Aufmerksamkeit kann max. 2-3 Sekunden aufrechterhalten werden. Die Kranke braucht ständige Pflege und Beobachtung.

Wegen der Wirkungslosigkeit und der Aussichtslosigkeit einer medikamentösen Behandlung wurde die Möglichkeit der Neurotransplantation in Erwägung gezogen. Nach der positiven Entscheidung der ethischen Kommission und nach Erhalt der schriftlichen Zustimmung der Verwandten wurde die Entscheidung über die Durchführung von intraparenchymatöser Autotransplantation mittels der aus dem Eigenknochenmark der Kranken gezüchteten Mesenchymstammzellen getroffen.

Während der Operation wurden in die Stirngegend (Kohers Punktprojektion) bilateral intraparenchymatös 7,5 Mio. Zellen und intravaskulär 7 Mio. Zellen eingeführt. Die Operation erfolgte ohne Komplikationen. Die Nähte wurden am 8. Tag entfernt.

Bereits an ersten Tagen nach der Operation nahmen die Erscheinungen der motorischen Enthemmung merkbar ab. Die Aggressivität verschwand so gut wie vollständig. Es wurde eine positive Dynamik in der Sprech-, Motorik- sowie in der Emotionssphäre festgestellt. Ein halbes Jahr nach der Autotransplantation der MSZ betrug der richtig gebrauchte Wortschatz über 30 Wörter. Die Kranke konnte ca. 80 Befehle richtig ausführen. Die Kranke bedient sich selbst, zieht sich an und aus, macht das Bett, benutzt selbstständig das WC. Sie kann sich selbst Kaffee zubereiten, das Geschirr vom Tisch aufräumen usw. Die Kranke ist lernfähig. Sie kann stundenlang mit großem Vergnügen Bilder in Büchern betrachten und nennt die Gegenstände darin richtig, sieht fern und spielt mit Puppen. Jedoch führt die Kranke richtige und gezielte Handlungen nur dann aus, wenn diese Handlungen für sie "interessant" sind. Es ist lediglich problematisch, sie etwas gegen ihren Wunsch machen zu lassen. Bei weiterer Beobachtung wird der Zustand der Kranken progressiv immer besser.

Beispiel 5 (nicht im Rahmen der Erfindung):

Kranker S., geb. 1995. Erblich unbelastet. Die Schwangerschaft der Mutter verlief mit Toxikose und Plazentaablösung. Die Geburt erfolgte vorzeitig (34 Wochen) mit Kaiserschnitt. Eine Stunde nach der Geburt kam es bei dem Kind zu einer Asphyxie. Es wurde in die Reanimationsabteilung gebracht, wo es einen Monat lang künstlich beatmet wurde. Danach erfolgte seine Entwicklung mit Verzögerung. Das Kind konnte seinen Kopf erst mit 6 Monaten halten und sich erst mit 11 Monaten setzen. Selbständiges Aufstehen erfolgte mit 13 Jahren. Mit 1,5 Jahren konnte das Kind lallen und mit 3 Jahren einzelne Wörter und kurze Wortverbindungen sprechen. Danach hörte es ganz auf zu sprechen. Die an das Kind gerichtete Rede wurde nur teilweise verstanden. Es wurde von Geburt an von einem Neurologen betreut. Diagnose: perinatale Enzephalopathie, Syndrom der motorischen Störungen. Das kranke Kind wird ständig medikamentös behandelt. Ab dem Alter von 6 Monaten wird es regelmäßig in verschiedenen Krankenhäusern behandelt (17 stationäre Aufnahmen). Verschiedene Diagnosen, von zerebraler Kinderlähmung (spastisch-asthenische Form) bis zum Syndrom des frühen Kinderautismus (zerebroorganische Genese) wurden erstellt. Das letzte Jahr hat das Kind ein Kinderrehabilitationszentrum besucht. Dabei wurden Reaktionen von Negativismus in Bezug auf Lehrer und Kinder festgestellt. Es wurde versucht, das Kind nach dem Lernprogramm für die 1. Klasse zu schulen, jedoch konnte es das Programm nicht bewältigen.

Aufnahmestatus: Formaler Kontakt, kein produktiver Kontakt möglich, sprechunfähig. Inadäquates Verhalten, inkonsequent, äußerst unbeständige Aufmerksamkeit. Der Kranke kann 8-10 einfache Befehle ausführen; kann sich nur teilweise bedienen, ist unreinlich. Der IQ ist auf dem Idiotie-Niveau. Keine Lernfähigkeit. Im Zusammenhang mit einem fehlenden Effekt nach erfolgter Behandlung und mit anhaltender Zustandsverschlimmerung wurde die Entscheidung über eine Neurotransplantation getroffen. Nach der Genehmigung der Ethik-Kommission und nach Erhalt der schriftlichen Zustimmung der Familienangehörigen wurde die Autotransplantation der Mesenchymstammzellen vorgenommen. Im Laufe der Autotransplantation wurden 8 Mio. Zellen bilateral in den Bereich der präfrontalen Großhirnrinde (präfrontaler Cortex) und gleichzeitig intravaskulär 53 Mio. Mesenchymstammzellen mit einer Geschwindigkeit von 6 Mio. Zellen pro Minute (intravenös) eingeführt.

Bereits an den ersten Tagen nach der Transplantation ließ die Aggression des Kranken gegenüber seiner Umgebung wesentlich nach. In der Folgezeit fing er an, kurze Wörter und Wortverbindungen richtig auszusprechen und zu gebrauchen. Der Bereich von Selbstbedienungsaktivitäten wurde wesentlich erweitert. Der Kranke begann, die Toilette selbständig zu benutzen, sich an- und ausziehen, das Bett zu machen usw. Die nachfolgende Katamnese zeugte von einer merklichen Verbesserung der Verbal- und kognitiven Funktionen. Der Kranke wurde lernfähig.

Beispiel 6 (nicht im Rahmen der Erfindung):

Kranker A., geb. 1992. Die Schwangerschaft bei der Mutter verlief unter Toxikose, die Geburt erfolgte mit einer ausgeprägten Asphyxie. Verzögerte Entwicklung. Es wurden progressive psychische und Sprechentwicklungshemmungen, Aggressivität beim Sprechen, unbeständige Aufmerksamkeit und kein Gedächtnis beim Lernen festgestellt.

IQ < 50. Nach dem Befund der Kommission über die Aussichtslosigkeit der medikamentösen Behandlung und dem Erhalt der schriftlichen Zustimmung der Verwandten wurde eine Transplantation der autologen Mesenchymstammzellen (MSZ) vorgenommen. Die MSZ wurden aus dem Knochenmark des Patienten mittels Gewebebehandlung abgeleitet, bis phänotypmäßig homogene Zellen gewonnen wurden. Diese Zellen wurden danach in vitro gezüchtet. Das Transplantat stellte einen modifizierten Stent dar, welcher als der mit MSZ gesättigte Träger diente. Insgesamt 42 Mio. Zellen wurden in die Hirnbereiche eingebracht.

Während der Operation wurde das Biotransplantat intraparenchymatös bilateral in die Stirngegend eingeführt. Die Nähte wurden 7 Tage später entfernt. Die Zustandsbesserung des Kranken wurde 2 Wochen später festgestellt. Es wird keine Aggressivität beobachtet. Die Beobachtung im Verlauf eines Jahres hat die Richtigkeit der Entscheidung über den chirurgischen Eingriff bestätigt. Der Kranke wurde lernfähig, sein Wortschatz wurde größer. Es wurde eine positive Dynamik in der Sprech-, Motorik- sowie in der Emotionssphäre festgestellt.

Beispiel 7 (nicht im Rahmen der Erfindung):

Kranke V., geb. 1996. Die Schwangerschaft der Mutter verlief mit Toxikose. Die Geburt erfolgte ohne besondere Vorkommnisse. Mit 2 Jahren wurde bei dem Kind die Diagnose "Hemmung der psychischen und emotionalen Entwicklung" und danach "Stereotypie bei Entwicklungshemmung" gestellt. Im Alter von 8 Jahren entsprach die Intelligenzentwicklung der eines Zweijährigen (Imbezillität). Nach Erhalt der Zustimmung der Eltern wurden der Kranken intravaskulär MSZ eingeführt. Die Einführung erfolgte intravenös mit einer Geschwindigkeit von 6 Mio. Zellen pro Minute und intraarteriell mit einer Geschwindigkeit von 2 Mio./Min. Die MSZ waren autolog. Sie wurden aus dem Knochenmark der Patientin gewonnen und in vitro gezüchtet. Die Gesamtmenge der eingebrachten Zellen betrug 54 Mio. Die Zellen waren ihrem Phänotyp nach homogen und lebensfähig.

Die Beobachtung der Kranken im Laufe des ersten Monats nach der MSZ-Einführung zeigte eine wesentliche Verbesserung ihres Zustands. Das ließ sich sowohl in ihrem Verhalten als auch in dem gezeigten Interesse für Bilderdemonstration in Büchern sowie in der beständigen Aufmerksamkeit feststellen. Das Kind wird weiter beobachtet. Es wurden weitere Fortschritte in der psychischen und Sprechentwicklung verzeichnet.

Beispiel 8 (nicht im Rahmen der Erfindung):

Kranke P., geb. 1989. Krankenhausaufenthalt mit Diagnose: Zerebrale Kinderlähmung, spastische Form mit ausgeprägter Entwicklungshemmung (Imbezillität). Am 24.02.05. wurde die Entnahme von Knochenmark mit nachfolgender Züchtung der autologen Mesenchymstammzellen und Lagerung der gewonnenen Kultur unter spezieller Tiefkühlung vorgenommen. Bei einer neuerlichen Krankenhauseinweisung (11.04.05) wurde die aufbereitete Kultur von MSZ entfrostet und der Kranken mittels intravaskulärer Injektion verpflanzt. Dabei betrug die Gesamtmenge der eingeführten MSZ 25 Mio. Zellen. Die Einführung erfolgte intraarteriell mit einer Geschwindigkeit von 2 Mio. Zellen pro Minute.

Vor der Transplantation wurde der Zustand folgendermaßen beschrieben: Vollständiges Fehlen des Sprechvermögens, nur teilweise Selbstbedienung, totale Enthemmung, Reizbarkeit, komplette Lernunfähigkeit und Bedarf an ständiger Pflege und Überwachung. Ein Jahr nach der Transplantation wurde bei einer Kontrollhospitalisierung eine wesentliche klinische Verbesserung festgestellt. Das Niveau der nonverbalen Intelligenz ist bedeutend gestiegen (p < 0,03). Obwohl immer noch kein Sprechen ausgebildet war, begann die Kranke, einzelne Silben und kurze Wörter auszusprechen. Der Wortschatz erreicht 50 Wörter, die Anzahl der richtig ausgeführten Befehle beträgt > 100. Sie kann stundenlang selbständig spielen oder fernsehen. Die Kranke wurde lernfähig. Sie begann, sich völlig selbständig zu bedienen. Es konnten beachtliche positive Änderungen gemäß den Ergebnissen der Überprüfung der sozialen und Lebensbedingungen erfasst werden (p < 0,01).

Beispiel 9 (nicht im Rahmen der Erfindung):

Kranke S., geb. 1995. Einlieferung ins Krankenhaus 2 Monate nach einem schweren Mixt- Schädelhirntrauma mit vorwiegender Schädigung der Basal-Stammstrukturen und Merkmalen von diffus axionaler Verletzung. Schwerer Zustand. Es wurden mehrere chirurgische Eingriffe vorgenommen. Danach erfolgte die Behandlung anhand der intravaskulären und intrazerebralen Einführung der lebensfähigen autologen MSZ, welche aus dem Knochenmark der Kranken gewonnen wurden. Die intravaskuläre Einführung der autologen MSZ erfolgte intraarteriell. Es wurden 20 Mio. MSZ, d.h. 0,7 Mio. MSZ pro 1 kg des Patientengewichts, eingeführt. Die Einführung der MSZ wurde mit einer Geschwindigkeit von 2 Mio./Min. ausgeführt. 20 Tage später wurde eine Operation zur Entfernung eines Hirnhämatoms durchgeführt und das Biotransplantat intrazerebral verpflanzt. Das Biotransplantat war der Träger mit autologen MSZ. Danach wurde die intravaskuläre Systemeinführung von MSZ vorgenommen. Dies erfolgte intravenös bei einer Geschwindigkeit von 6 Mio./Min.

2,5 Monate nach der Behandlung unter Einsatz von MSZ wurde ein positiver Zustand der Patientin erreicht. Die Vitalfunktionen wurden stabil. Der positive Effekt der Behandlung blieb erhalten, was durch die 8 Monate später erfolgte Kontrolle bestätigt wurde.

Beispiel 10 (nicht im Rahmen der Erfindung):

Kranke P., geb. 1947. Laut Anamnese - akute Kreislaufstörung, zerebrovaskuläre Störung. Die Kranke wurde 3,5 Jahre nach dem Trauma ins Krankenhaus eingeliefert. Die Behandlung erfolgte unter Einsatz von intravaskulärer und intrazerebraler Einführung der lebensfähigen autologen MSZ, welche aus dem Knochenmark der Kranken gewonnen wurden. Die autologen Zellen für die Transplantation wurden nach ihrer Züchtung (Speicherung) in vitro genommen. Befund des EEG vor der Operation: Interhemisphären-Asymmetrie. In der rechten Hemisphäre werden leichte allgemeine Hirnveränderungen mit residualem organischem Charakter und in der linken Hemisphäre wesentliche Veränderungen festgestellt. Der Herd der organischen Aktivität befand sich in der linken Schläfengegend. Die Operation der osteoplastischen Schädeltrepanation wurde in der linken Stirn-Scheitel-Schläfengegend vorgenommen. Es wurde eine Dränage der Zysten der linken Hemisphäre des Gehirns vorgenommen. Danach wurde eine intrazerebrale Injektion des Biotransplantats durchgeführt. Das Biotransplantat bestand aus den autologen MSZ und dem autologen Blutserum. Die MSZ wurden bei etwa 7-12 Mio. pro Brodmann-Zone genommen.

8 Tage später wurde mit intravaskulärer Systemeinführung der autologen MSZ mittels intravenöser Injektion bei einer Geschwindigkeit von 6 Mio./Min. begonnen. Die einmalige Einführung umfasste 12 Mio. MSZ.

Eine erneute Krankenhauseinweisung erfolgte 6 Monate später. Im Vergleich zum EEG nach der ersten MSZ-Einführung wurde eine geringe Abnahme der allgemeinen und der Herd-Hirnstörungen festgestellt. Es wurde eine Behandlung einschließlich der intravaskulären Systemeinführung von MSZ (intravenös) vorgenommen. Die Gesamtmenge der Zellen betrug 36 Mio. MSZ.

Infolge der Behandlung wurde ein beständiger, befriedigender Zustand der Kranken erreicht.

**Patentansprüche**

1. Verfahren zur Gewinnung einer Population von Mesenchymstammzellen (MSZ), die eine phänotypmäßige Homogenität von 87 bis 99,9% bezüglich der Zellmarkern CD 44+, CD 90+, CD 105+, CD 106+, CD 45-, CD 34- aufweist, wobei die Mesenchymstammzellen aus Knochenmark von Säugetieren gewonnen werden, und das Verfahren die Ableitung der MSZ anhand der Zentrifugierung und Züchtung im Kulturmedium einschließt, **dadurch gekennzeichnet, dass**

   a) die lebensfähigen Zellen an der Grenze der Medien DMEM-Ficoll in der Verarbeitung des Ausgangsmaterials nach einer Zentrifugierung von 25 bis 60 Minuten der aufs Ficoll aufgeschichteten Suspension der Zellen des heparinisierten Knochenmarkpunktats im DMEM bei einer Geschwindigkeit von 1000 bis 2000 U/Min abgeleitet werden,
   b) die Zellen mit einer Dichte von 75-200 Zellen pro 1 cm$^2$ ausgesät werden
   c) die Züchtung der Zellmasse aus den gewonnen MSZ im genormten Geschirr vorgenommen wird unter Verwendung von DMEM-Medium, das 15 - 22% FBS zusätzlich enthält,

   wobei die Inkubationsdauer 18 bis 52 Stunden beträgt und
   die Zentrifugation bei einer Temperatur von 18 bis 23 °C vorgenommen wird.

2. Verfahren nach Anspruch 1,
   **dadurch gekennzeichnet**
   **dass** die Zellmasse in einer Menge von 1,5 - 3 x 10$^6$ bis 1,5 - 3 x 10$^7$ Zellen pro 1 ml des Ausgangsknochenmarks im Laufe von drei Passagen gewonnen wird.

3. Verfahren nach Anspruch 1,
   **dadurch gekennzeichnet**
   **dass** die Zentrifugierung im Ficoll mit einer Dichte von 1,077 g/ml oder 1,115 g/ml vorgenommen wird.

**Claims**

1. A method of obtaining a population of mesenchymal stem cells (MSCs) having a phenotypic homogeneity of 87-99.9 % with respect to the cell markers CD 44+, CD 90+, CD 105+, CD 106+, CD 45-, CD 34-, the mesenchymal stem cells being obtained from bone marrow of mammals, the method including the derivation of the MSCs based on centrifugation and cultivation in a culture medium,
   **characterized in that**

   a) the viable cells are derived at the boundary of the media DMEM-Ficoll in the processing of the initial material after a centrifugation for 25-60 minutes of the suspension of the cells of the heparinised bone marrow aspirate layered on the Ficoll in the DMEM at a speed of 1000-2000 rpm,
   b) the cells are seeded at a density of 75-200 cells per 1 cm$^2$
   c) the cultivation of the cell mass from the obtained MSCs is performed in standardized dishes using DMEM medium additionally comprising 15-22% FBS,

   wherein the incubation period is 18-52 hours, and
   the centrifugation is performed at a temperature of 18-23°C.

2. The method according to claim 1,
   **characterized in that**
   the cell mass is obtained in an amount of 1.5-3 $\times$ 10$^6$ to 1.5-3 $\times$ 10$^7$ cells per 1 ml of the initial bone marrow in the course of three passages.

**3.** The method according to claim 1,
**characterized in that**
the centrifugation is performed in Ficoll having a density of 1.077 g/l or 1.115 g/l.


**Revendications**

**1.** Procédé d'obtention d'une population de cellules souches mésenchymateuses (CSM), qui présente une homogénéité phénotypique de 87 à 99,9 % pour ce qui concerne les marqueurs cellulaires CD 44+, CD 90+, CD 105+, CD 106+, CD 45-, CD 34-, les cellules souches mésenchymateuses étant obtenues à partir de la moelle osseuse de mammifères, et le procédé comprenant la dérivatisation des CSM par centrifugation et culture dans un milieu de culture, **caractérisé en ce que**

a) on dérivatise les cellules vivantes au niveau de la frontière entre les milieux DMEM et Ficoll, lors du traitement de la matière de départ après une centrifugation de 25 à 60 minutes de la suspension, appliquée sur Ficoll, des cellules de la ponction de moelle osseuse héparinisée dans du DMEM à une vitesse de 1000 à 2000 tr/min,
b) on ensemence les cellules pour une densité de 75 à 200 cellules par cm$^2$,
c) on procède à la culture de la masse cellulaire provenant des CSM obtenues, dans une vaisselle normalisée, par utilisation d'un milieu DMEM qui contient en outre 15 à 22 % de FBS,

la durée de l'incubation étant de 18 à 52 heures, et la centrifugation étant mise en oeuvre à une température de 18 à 23°C.

**2.** Procédé selon la revendication 1, **caractérisé en ce que** la masse cellulaire est obtenue dans le cadre de trois passages en une quantité de 1,5 à 3 x 10$^6$ à 1,5 à 3 x 10$^7$ cellules par ml de la moelle osseuse de départ.

**3.** Procédé selon la revendication 1, **caractérisé en ce que** la centrifugation dans le Ficoll est réalisée pour une masse volumique de 1,077 g/ml ou 1,115 g/ml.

**Fig. 1**

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005121317 A

- RU 2219937

**In der Beschreibung aufgeführte Nicht-Patentliteratur**

- *Tierzellenkultur. Methoden. Herausgegeben von R. Freshni. Moskau. Mir.,* 1989
- *Bone,* 1995, vol. 13, 81-95
- **MAJUMDAR M. K. et al.** *J. Cell. Physiol.,* Oktober 2000, vol. 185 (1), 98-106
- *J. Cell. Physiol.,* 2000, vol. 185, 98-106

- **A.G. RUMYANTSEV ; A.A. MASCHAN.** Transplantation der hämopoetischen Stammzellen. *Medizinische Infoagentur. M.,* 2003, 912
- **G.E. SUKHAREVA.** Vorlesungen über Psychologie des Kinderalters. *M. Medizin.,* 1974, 320